# FASCICULE DE BREVET EUROPEEN

(11) **EP 3 768 152 B1**
(45) Date de publication et mention de la délivrance du brevet: **30.03.2022**
(21) Numéro de dépôt: 19712754.1
(22) Date de dépôt: 20.03.2019
(51) Int. Cl.: A61B 5/00, A61B 5/021, A61B 5/024, A61B 5/0255, A61B 5/16, A61B 5/0295

(54) **SYSTEME POUR LE SUIVI DU STRESS CHEZ UN INDIVIDU**
SYSTEM ZUR ÜBERWACHUNG VON STRESS BEI EINER PERSON
SYSTEM FOR MONITORING STRESS IN AN INDIVIDUAL

(30) Priorité: 21.03.2018 FR 1852442
(43) Date de publication de la demande: 27.01.2021
(73) Titulaire: Axelife, 44460 Saint Nicolas de Redon (FR)
(72) Inventeur: HALLAB, Magid, 44460 SAINT NICOLAS DE REDON (FR)
(74) Mandataire: Ermeneux, Bertrand
(86) Numéro de dépôt international: PCT/EP2019/056962
(87) Numéro de publication internationale: WO 2019/180083

(56) Documents cités:
- US-A1- 2003 036 685
- US-A1- 2013 324 859
- US-A1- 2017 238 818
- MILLASSEAU SANDRINE C ET AL: "Contour analysis of the photoplethysmographic pulse measured at the finger", JOURNAL OF HYPERTENSION, LIPPINCOTT WILLIAMS & WILKINS, LTD, GB, vol. 24, no. 8, 1 août 2006 (2006-08-01), pages 1449-1456, XP002673731, ISSN: 0263-6352, DOI: 10.1097/01.HJH.0000239277.05068.87 cité dans la demande

## Description

Le domaine de l'invention est celui des équipements non invasifs de mesure de paramètres physiologique d'un individu humain.

Plus précisément, l'invention concerne un système pour la détermination d'un facteur de stress d'un individu humain.

Dans le domaine de l'invention, il a été montré que le stress peut provoquer chez certains individus des désagréments tels que des pertes de conscience momentanées, des évanouissements, des malaises ou encore des pertes de reflexes ou de capacités motrices.

Outre ces désagréments, le stress peut engendrer des conséquences beaucoup plus graves parmi lesquelles on peut citer les infarctus, les accidents vasculaires cérébraux et les troubles cognitifs.

Il est donc important de surveiller l'apparition du stress afin d'éviter de tels effets sur la santé.

Pour cela il est connu de surveiller certains paramètres physiologiques comme la pression artérielle ou encore la vitesse de propagation de l'onde de pouls par exemple.

On connait ainsi du document US2017/238818 A1 une technique de calcul de la pression artérielle basée entre autres sur un algorithme de Pan-Tompkins, utilisant l'aire entre le pic systolique et l'encoche dichrotique, l'aire entre l'encoche dichrotique et le pic diastolique et des caractéristiques non linéaires de l'onde.

Le document US2003/036685 A1 propose par ailleurs une technique de détermination de la pression artérielle moyenne à partir de l'onde systolique.

De nouvelles méthodes ont été développées pour déterminer de nouveaux facteurs de stress, comme par exemple le calcul de la rigidité artérielle qui influence la distribution du sang dans le corps.

En effet, une rigidité artérielle trop importante engendre une surpression dans les artères périphériques, cette surpression pouvant notamment être la cause des désagréments et des conséquences précités.

Parmi les paramètres physiologiques influents sur le stress, le suivi de l'évolution du pouls, de la pression artérielle, de la pression centrale et de la rigidité artérielle permettent d'obtenir un bon suivi de l'état de stress artériel d'un individu dans le temps.

Pour mesurer ces paramètres physiologiques, des machines d'analyse sont utilisées. Ces machines d'analyse, qui mettent en œuvre des procédés complexes pour le suivi de ces paramètres physiologiques, nécessitent la présence d'un personnel médical qualifié pour leur utilisation et pour la lecture des résultats d'analyses.

Dès lors, ces machines d'analyse sont utilisées dans des centres d'analyses dans lesquels les individus, également appelés patients, se rendent pour procéder à la détermination de leur état de stress.

Toutefois, de telles analyses ne sont pas le reflet de la réalité en ce qu'elles ne sont réalisées que de manière ponctuelle, c'est-à-dire à plusieurs jours d'intervalle, lorsque l'individu se trouve dans un centre d'analyse.

Par ailleurs, pour réaliser ces analyses, l'individu doit se rendre disponible afin d'aller au centre d'analyse, ce qui n'est pas évident pour des individus ayant un emploi du temps chargé, notamment pour des professionnels occupant des postes à responsabilités ou effectuant des déplacements fréquents.

Or, il est réputé que les emplois du temps chargés et/ou les déplacements fréquents augmentent le risque de stress et les désagréments qui y sont liés. Les difficultés à suivre une alimentation équilibrée et variée et le changement fréquent de logement peuvent notamment expliquer l'augmentation du risque de stress.

De plus, lorsque l'individu se trouve dans un centre d'analyse, il est plongé dans des ambiances généralement calmes et paisibles qui ont pour but de le détendre. Ainsi, les analyses ne sont pas effectuées dans des conditions réelles dans lesquelles l'individu évolue généralement. Ces analyses sont donc difficilement exploitables par les professionnels de santé pour déterminer les facteurs de stress ou encore la résistance au stress de l'individu.

Dès lors, un professionnel de santé pourrait être amené à empêcher un individu d'exercer sa profession alors qu'il résiste au stress qui y est lié ou, au contraire, ne pas empêcher un individu d'exercer sa profession alors que le stress lié à sa profession peut entraîner une variabilité trop importante de ses paramètres physiologiques et donc présenter un risque important pour la santé de l'individu.

La variabilité s'entend de l'évolution des paramètres physiologiques dans le temps.

L'invention a notamment pour objectif de palier les inconvénients de l'art antérieur.

Plus précisément, l'invention a pour objectif de fournir un système permettant de suivre l'évolution de paramètres physiologiques indicateurs de stress de manière simple et régulière.

L'invention a également pour objectif de proposer un tel système pouvant être utilisé par un individu sans avoir recours à un professionnel de la santé.

L'invention a en outre pour objectif de proposer un tel système qui puisse être utilisé sans contraintes pour l'individu, notamment sans nécessiter un déplacement dans un centre d'analyse, ni l'intervention d'un opérateur ou de l'individu pour réaliser les mesures.

Ces objectifs, ainsi que d'autres qui apparaîtront par la suite, sont atteints grâce à l'invention qui a pour objet un système comprenant :
- un dispositif destiné à être solidarisé au corps d'un individu, le dispositif comprenant des moyens de mesure intégrant au moins un capteur de type photopléthysmographique destiné à acquérir des données brutes instatanées ;
- une première unité de calcul d'une valeur d'onde de pouls instantanée à partir des données brutes instantanées du capteur photopléthysmographique ;
- une unité de stockage des valeurs d'onde de pouls calculées ;
- une unité de traitement des valeurs d'onde de pouls stockées, comprenant :
   - des moyens d'identification d'une période pour les valeurs d'onde de pouls stockées ;
   - une deuxième unité de calcul pour calculer, pour chaque période identifiée :
      - une différence d'amplitude et une différence de temps entre un premier point correspondant à un pic systolique et un deuxième point correspondant à un pic diastolique,
      - un instant médian entre le premier point et le deuxième point,
- une troisième unité de calcul pour calculer, à partir de la différence d'amplitude, de la différence de temps et de l'instant médian, au moyen d'une analyse statistique multivariée, pas à pas avec calibration périphérique, les trois paramètres physiologiques suivants :
   - un indice de rigidité artérielle ;
   - une pression artérielle ;
   - une pression centrale,
- une quatrième unité de calcul d'une valeur de variation dans le temps desdits trois paramètres physiologiques à deux instants T différents.

Grâce au dispositif portable, le suivi du stress de l'individu peut être fait de manière régulière sans que ce dernier ne s'en aperçoive. En effet, l'individu peut poursuivre ses différentes activités sans avoir à penser à s'arrêter pour le contrôle de son stress.

Ainsi, les mesure effectuées par le dispositif sont faites dans l'environnement normal de l'individu, contrairement au mesures impliquant de déplacer l'individu dans des lieux spécialisées, ou de devoir être confronté à un professionnel de la santé, comme dans l'art antérieur.

Avantageusement, le système comprend également un dispositif communicant externe de type ordiphone reliée au dispositif.

Le dispositif communicant externe peut notamment servir d'interface de contrôle du dispositif ou de moniteur d'affichage d'informations provenant du dispositif.

Par ailleurs, le dispositif communicant externe peut notamment servir à émettre une alerte à destination d'un professionnel de la santé ou d'un proche de l'individu portant le dispositif. Ainsi, en cas d'alerte, au moins une personne peut être informée pour essayer de contacter l'individu afin de :
- constater l'inconscience de l'individu et demander l'intervention de secours,
- ou, au contraire, se rassurer sur la conscience de l'individu et lui conseiller de consulter un professionnel de santé ou de se reposer.

Selon un premier mode préféré de réalisation, les unités de calcul, l'unité de traitement et l'unité de stockage sont intégrées au dispositif.

Ainsi, le dispositif peut être autonome pour réaliser le suivi du stress de l'individu.

Selon un deuxième mode préféré de réalisation, les unités de calcul, l'unité de traitement et l'unité de stockage sont intégrées au dispositif communicant externe.

Ainsi, le système peut bénéficier d'une puissance de calcul accrue pour réaliser le suivi du stress de l'individu. Par ailleurs, cela permet notamment d'alléger le dispositif de sorte que l'individu ne ressente pas que le dispositif est solidarisé à son corps.

Selon une caractéristique avantageuse, le dispositif comprend des moyens sensoriels.

Les moyens sensoriels permettent au dispositif de communiquer avec l'individu par le biais de ses sens pour l'alerter lorsque son stress est trop important et présente un risque pour sa santé par exemple.

De préférence, les moyens sensoriels comprennent au moins un organe parmi :
- un organe lumineux ;
- un organe vibratoire ;
- un organe sonore.

Ces organes peuvent notamment n'être perceptibles que pour l'individu de sorte que l'alerte sur son état de stress ne soit connue que de lui.

Avantageusement, le dispositif comprend un actionneur relié aux moyens sensoriels et actionnable par l'individu pour désactiver les moyens sensoriels.

L'action de l'individu sur l'actionneur permet notamment de savoir que l'individu est conscient. Au contraire, lorsque l'alerte est sonore et que l'individu est inconscient, toute personne passant près de l'individu inconscient peut alors être alerté à son tour et contacter les secours ou porter lui-même les premiers secours à l'individu inconscient.

Selon un autre mode préféré de réalisation, le dispositif comprend également au moins l'un parmi :
- un capteur de température ;
- un capteur d'oxygène ;
- un accéléromètre ;
- un capteur d'hydratation.

Ainsi, le système, et plus particulièrement le dispositif, permet d'alerter un personnel médical de la position de l'individu en cas de chute, ou de stress important nuisibles pour sa santé.

D'autres caractéristiques et avantages de l'invention apparaîtront plus clairement à la lecture de la description suivante d'un mode de réalisation préférentiel de l'invention, donné à titre d'exemple illustratif et non limitatif, et des dessins annexés parmi lesquels :
- la figure 1 est une représentation schématique d'un système selon un premier mode de réalisation de l'invention ;
- la figure 2 est une représentation schématique d'un système selon un deuxième mode de réalisation de l'invention ;
- la figure 3 est une modélisation d'une période d'une onde de pouls.

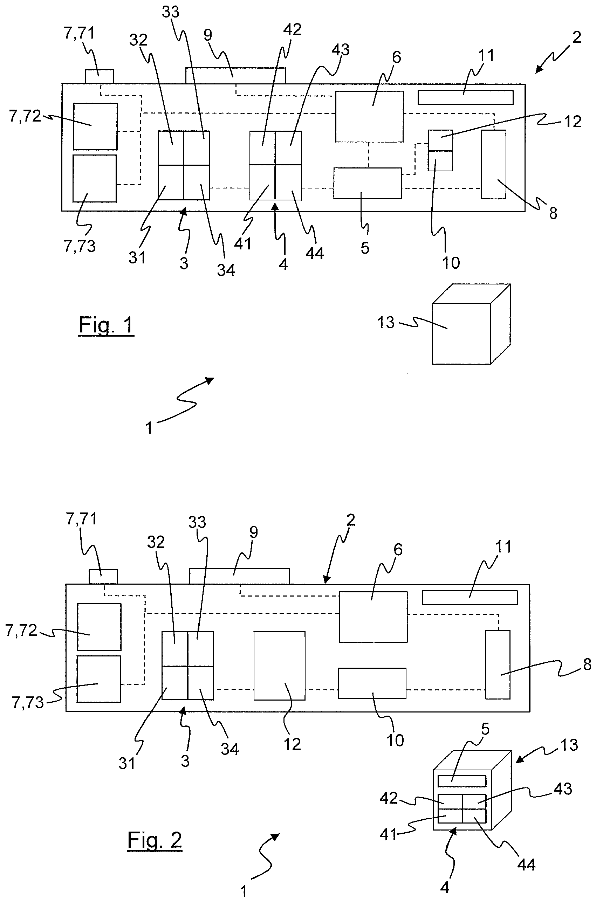

Tel qu'illustré schématiquement sur les figures 1 et 2, un système 1 selon l'invention permet de suivre l'évolution de paramètres physiologiques d'un individu pour l'alerter d'un stress trop important pouvant engendrer un risque pour sa santé.

Le système 1 comprend un dispositif 2 destiné à être solidarisé au corps d'un individu.

Le dispositif 2 se présente par exemple sous la forme d'un bracelet destiné être positionné autour du poignet d'un individu ou d'un patch destiné à être rapporté sur un vêtement moulant porté par l'individu.

Comme représenté sur les figures 1 et 2, le dispositif 2 comprend des moyens de mesure 3 intégrant au moins un capteur 31 de type photopléthysmographique destiné à acquérir des données brutes instantanées. Le capteur 31 photopléthysmographique (PPG) est une photodiode qui utilise des ondes lumineuses traversant le corps et notamment une artère, dans l'infrarouge, le rouge, le bleu ou le vert.

Le dispositif 2 est avantageusement portatif, c'est-à-dire qu'il est destiné à être solidarisé au corps de l'individu dont on veut suivre le stress.

Le système 1 comprend également :
- une première unité de calcul 41 d'une valeur d'onde de pouls instantanée à partir des données brutes instantanées du capteur 31 photopléthysmographique ;
- une unité de stockage 5 des valeurs d'onde de pouls calculées ;
- une unité de traitement 42 des valeurs d'onde de pouls stockées, comprenant :
   - des moyens d'identification d'une période pour les valeurs d'onde de pouls stockées ;
   - une deuxième unité de calcul pour calculer, pour chaque période identifiée :
      - une différence d'amplitude et une différence de temps entre un premier point correspondant à un pic systolique et un deuxième point correspondant à un pic diastolique,
      - un instant médian entre le premier point et le deuxième point,
- une troisième unité de calcul 43 pour calculer, à partir de la différence d'amplitude, de la différence de temps et de l'instant médian, au moyen d'une analyse statistique multivariée, pas à pas avec calibration périphérique, les trois paramètres physiologiques suivants :
   - un indice de rigidité artérielle ;
   - une pression artérielle ;
   - une pression centrale,
- une quatrième unité de calcul 44 d'une valeur de variation dans le temps desdits trois paramètres physiologiques à deux instants de mesure différents.

Les unités de calcul 41, 43, 44 et l'unité de traitement 42 sont regroupés dans un calculateur 4 et permettent, par l'utilisation d'une méthode de calcul telle que décrite dans la publication « Contour analysis of the photoplethysmographic pulse measured at the finger. Journal of hypertension » par Millasseau, S. C., Ritter, J. M., Takazawa, K., & Chowienczyk, P. J., de calculer l'indice de rigidité artérielle, la pression artérielle et la pression centrale.

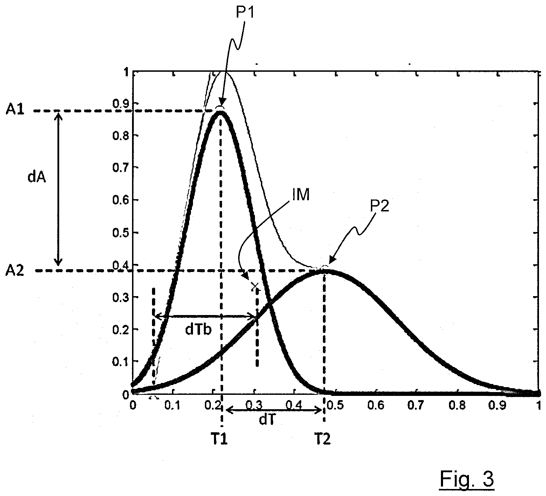

Plus précisément, en référence à la figure 3, après identification d'une période pour les valeurs d'onde de pouls stockées, l'unité de traitement 42 détermine, sur cette période identifiée, un premier point P1 correspondant à un pic systolique et un deuxième point P2 correspondant à un pic diastolique.

Le point P1 présente une amplitude A1 intervenant à un instant T1 et le point P2 présente une amplitude A2 intervenant à un instant T2.

A partir des points P1 et P2, l'unité de traitement 42 calcule pour chaque période identifiée :
- une différence d'amplitude dA et une différence de temps dT entre le premier point P1 et le deuxième point P2,
- un instant médian IM entre le premier point P1 et le deuxième point P2.

Ensuite, la troisième unité de calcul 43 calcule, à partir de la différence d'amplitude dA, de la différence de temps dT et de l'instant médian IM, les trois paramètres physiologiques suivants :
- un indice de rigidité artérielle ;
- une pression artérielle ;
- une pression centrale.

La détermination des trois paramètres physiologiques est réalisée au moyen d'une analyse statistique multivariée, pas à pas avec calibration périphérique, c'est-à-dire une analyse statistique à plusieurs variables utilisant les lois de probabilité.

La rigidité artérielle peut également être calculée selon la formule SI = h/T_{T}, (avec SI en mètres par secondes), où h est la taille de l'individu (en mètres) et T_{T} est la différence de temps (en secondes) premier point P1 et le deuxième point P2.

En outre, les unités de calcul 41, 43, 44 et l'unité de traitement 42 peuvent également permettre de calculer la tension, le pouls et l'arythmie cardiaque, c'est-à-dire un trouble du rythme cardiaque de l'individu porteur du dispositif 2, sur une période donnée.

De préférence, les moyens de mesure 3 comprennent également un capteur 32 de température, un capteur 33 d'oxygène et un capteur 34 d'hydratation.

Tout comme le capteur 31 photopléthysmographique, le capteur 32 de température, le capteur 33 d'oxygène et le capteur 34 d'hydratation sont reliés aux unités de calcul 41, 43, 44 et à l'unité de traitement 42 du calculateur 4.

Le capteur 32 de température permet de connaître la température de l'individu, de sorte quel le calculateur 4 puisse détecter une infection par une augmentation de la température combinée à une augmentation du pouls et une chute de tension de l'individu porteur du dispositif 2.

Le dispositif 2 comprend en outre :
- des moyens d'alerte 6 ;
- des moyens sensoriels 7 ;
- des moyens de communication 8 ;
- au moins un actionneur 9 ;
- un accéléromètre 10 ;
- une batterie 11,
- un module de géolocalisation 12.

Toutes les données acquises par les moyens de mesure 3, sont alors stockées dans l'unité de stockage 5 pour être utilisées par l'unité de traitement 42 comme expliqué ci-après.

L'accéléromètre 10 est couplé au calculateur pour détecter un changement brutal de hauteur, correspondant par exemple à une chute de l'individu portant le dispositif 2.

Le module de géolocalisation 12, quand à lui, permet de connaître la position du dispositif 2 et donc la position de l'individu qui le porte.

Les moyens d'alerte 6 sont reliés au calculateur 4, aux moyens sensoriels 7, ainsi qu'à l'actionneur 9.

Les moyens sensoriels 7 sont par exemple :
- un organe lumineux 71 ;
- un organe vibratoire 72 ;
- un organe sonore 73.

Bien que les exemples illustrés montrent un dispositif 2 comprenant à la fois un organe lumineux 71, un organe vibratoire 72 et un organe sonore 73, le dispositif 2 pourrait ne comporter qu'un seul ou deux de ces organes.

L'actionneur 9 est en l'espèce un bouton actionnable par l'individu porteur du dispositif 2.

La batterie 11 est une batterie rechargeable du genre des batteries couramment utilisées pour les téléphones mobiles par exemple.

Avantageusement, le dispositif 2 peut également comprendre un film photoélectrique (par exemple d'un micron d'épaisseur) collé sur le dispositif 2 et relié à la batterie 11 pour assurer son chargement.

D'autres moyens tels qu'un connecteur peuvent être prévus pour recharger la batterie 11. Les connecteurs peuvent également permettre une liaison entre le dispositif 2 et un ordinateur, notamment pour un échange de données entre l'ordinateur et l'unité de stockage 5.

Par ailleurs, la batterie 11 peut être une pile qu'il est possible de changer à la manière des piles d'une montre ou d'un objet électronique ou d'être rechargée par un dispositif externe ad hoc.

Les moyens de communication 8 sont reliés aux moyens d'alerte 6 et au calculateur 4 et sont destinés à communiquer avec un dispositif communicant externe 13.

Le dispositif communicant externe 13 est par exemple un ordiphone, un téléphone portable, un serveur informatique d'un centre de surveillance médicale ou de téléassistance, ou encore un téléphone d'un proche.

Toutefois, bien qu'un seul dispositif communicant externe 13 est représenté sur les figures 1 et 2, les moyens de communication 8 peuvent échanger des données avec plusieurs dispositifs communicants externes 13.

Par exemple, le dispositif 1 peut être couplé à la fois à un ordiphone de l'individu portant le dispositif 2, à un serveur informatique distant contrôlé par un professionnel de la santé pour effectuer un suivi de la rigidité artérielle SI de l'individu, et à un téléphone portable, tel que celui d'un proche à alerter si la santé de l'individu est menacée.

Pour échanger avec le ou chaque dispositif communicant externe 13, les moyens de communication 8 utilisent des protocoles ou des réseaux de communications existants tels que par exemple le Bluetooth (marque déposée), le Wi-Fi, la 3G ou la 4G par exemple, ou tout autre protocole permettant un échange d'informations.

En outre, le dispositif 2 peut également comprendre un écran (non représenté) permettant d'afficher des informations en temps réel telles que l'heure ou encore la date.

Selon le premier mode de réalisation illustré sur la figure 1, le dispositif 2 intègre le calculateur 4 et l'unité de stockage 5. Le dispositif communicant externe 13 peut notamment servir d'interface pour contrôler valeur de variation dans le temps desdits trois paramètres physiologiques, de la température, la température de l'individu ou toute autre donnée provenant des moyens de mesure.

Selon le deuxième mode de réalisation illustré sur la figure 2, le calculateur 4 et l'unité de stockage 5 sont des éléments du dispositif communicant externe 13. Dans ce cas, les données des moyens de mesure 3 sont transmises directement au calculateur 4 via les moyens de communication 8 du dispositif 2.

En fonctionnement, lorsque le calculateur 4 détecte, grâce à la quatrième unité de calcul 44, une valeur de variation dans le temps desdits trois paramètres physiologiques à deux instants T différents, alors les moyens d'alerte 6, grâce à leur configuration, actionnent les moyens sensoriels 7 de sorte à alerter l'individu d'un risque pour sa santé.

A titre indicatif, les moyens sensoriels 7 sont actionnés dès lors que la valeur de variation dans le temps de l'un au moins des paramètres physiologiques dépasse 10%.

Pour l'actionnement des moyens sensoriels 7, la valeur de variation est toutefois paramétrable en fonction de l'individu qui porte le dispositif 2, de sorte que le système 1 soit dédié à un individu en particulier et que son analyse des risques de stress soit précise. Ainsi, la valeur de 10% précitée peut être supérieure ou inférieure selon l'individu porteur du dispositif 2.

Si l'individu porteur du dispositif 2 est conscient, il peut alors désactiver les moyens sensoriels 7 en agissant sur l'actionneur 9.

En revanche, si l'individu porteur du dispositif 2 n'est pas conscient, alors les moyens sensoriels 7 restent actifs et les moyens de communication 8 sont utilisés pour communiquer avec au moins un dispositif communicant externe 12 afin d'alerter un proche de l'individu ou un professionnel de santé. A cette dernière occasion, les coordonnées de localisation de l'individu, déterminées par le module de géolocalisation 12, sont transmises pour permettre l'intervention d'une équipe de secours.

Par ailleurs, à intervalle de temps régulier, les moyens de communication 8 peuvent être utilisés pour transmettre à un serveur distant des relevés de la valeur des trois paramètres physiologiques surveillés et éventuellement des autres paramètres physiologiques détectés ou calculés.

En outre, les moyens de communication 8 peuvent également être utilisés pour activer les moyens sensoriels 7 si un professionnel de la santé estime que la valeur de variation dans le temps de l'un au moins des paramètres physiologiques est inquiétante sans pour autant atteindre les 10% précités (ou un seuil défini en fonction de l'individu porteur du dispositif 2).

En outre, le calculateur 4 est également paramétré, à l'aide des moyens de mesure 3, pour permettre la détection ou le calcul d'un ou plusieurs facteurs parmi :
- une chute de l'individu ;
- une arythmie cardiaque ;
- d'une baisse de l'oxymétrie ;
- une augmentation anormale de la fréquence cardiaque.

Lorsque l'un de ces facteurs au moins est détecté, alors les moyens d'alerte 6 peuvent commander les moyens sensoriels 7 de sorte à émettre un signal d'alerte à destination de l'individu et/ou commander les moyens de communication 8 de sorte à envoyer des données d'alerte à destination d'un ou de chaque dispositif communicant externe 13.

Pour les facteurs ci-dessus, des seuils d'alerte, donnés à titre d'exemple ci-dessous, sont définis :
- augmentation de la fréquence cardiaque de plus de 20% par rapport à la valeur précédente ;
- baisse de l'oxymétrie de plus de 5%.

Différents niveaux d'alerte peuvent être définis comme par exemple :
- un premier niveau si un seul facteur ou si une valeur de variation dans le temps de l'un au moins des paramètres physiologiques dépassant 10% sont identifiées ;
- un deuxième niveau si deux facteurs ou valeurs de variation dans le temps de l'un au moins des paramètres physiologiques dépassant 10% ;
- un troisième niveau si trois facteurs et/ou valeurs de variations sont identifiés.

Lorsque deux facteurs et/ou valeurs de variation au moins sont identifiés simultanément (correspondant à une alerte de deuxième niveau ou de troisième niveau), le calculateur 4 est configuré pour refaire une analyse des données afin de vérifier que les facteurs identifiés et/ou valeurs de variation sont bien existants et que la santé de l'individu est réellement menacée.

A titre indicatif, l'acquisition de données brutes par les moyens de mesure 3, notamment par le capteur 31 photopléthysmographique, est faite quatre ou cinq fois par jour.

Le dispositif 2 qui vient d'être décrit offre comme principal avantage d'être portable par un individu, ce qui permet de réaliser un suivi continu de l'évolution de son état de stress, notamment dans le but de prévenir d'un risque imminent pour sa santé.

L'individu porteur du dispositif 1 peut ainsi être alerté, de manière quasi instantanée, lorsqu'il est soumis à un stress important pouvant nuire à sa santé, pour arrêter l'activité qu'il réalise afin de se reposer de manière à faire descendre le niveau de stress et réduire le risque pour sa santé, ou pour consulter un professionnel de santé.

## Revendications

1. Système (1) comprenant :
- un dispositif (2) destiné à être solidarisé au corps d'un individu, le dispositif (2) comprenant des moyens de mesure (3) intégrant au moins un capteur (31) de type photopléthysmographique destiné à acquérir des données brutes instantanées ;
- une première unité de calcul (41) d'une valeur d'onde de pouls instantanée à partir des données brutes instantanées du capteur (31) photopléthysmographique ;
- une unité de stockage (5) des valeurs d'onde de pouls calculées ;
- une unité de traitement (42) des valeurs d'onde de pouls stockées, comprenant :
- des moyens d'identification d'une période pour les valeurs d'onde de pouls stockées ;
- une deuxième unité de calcul pour calculer, pour chaque période identifiée :
- une différence d'amplitude et une différence de temps entre un premier point correspondant à un pic systolique et un deuxième point correspondant à un pic diastolique,
- un instant médian entre le premier point et le deuxième point,
- une troisième unité de calcul (43) pour calculer, à partir de la différence d'amplitude, de la différence de temps et de l'instant médian, au moyen d'une analyse statistique multivariée, pas à pas avec calibration périphérique, les trois paramètres physiologiques suivants :
- un indice de rigidité artérielle ;
- une pression artérielle ;
- une pression centrale,
- une quatrième unité de calcul (44) d'une valeur de variation dans le temps desdits trois paramètres physiologiques à deux instants T différents.

2. Système (1) selon la revendication 1, **caractérisé en ce qu'**il comprend également un dispositif communicant externe (13) de type ordiphone relié au dispositif (2).

3. Système (1) selon la revendication 1 ou la revendication 2, **caractérisé en ce que** les unités de calcul (41, 43, 44), l'unité de traitement (42) et l'unité de stockage (5) sont intégrées au dispositif (2).

4. Système (1) selon la revendication 2, **caractérisé en ce que** les unités de calcul (41, 43, 44), l'unité de traitement (42) et l'unité de stockage (5) sont intégrées au dispositif communicant externe (13).

5. Système (1) selon la revendication précédente, **caractérisé en ce que** le dispositif (2) comprend des moyens sensoriels (7).

6. Système (1) selon la revendication précédente, **caractérisé en ce que** les moyens sensoriels (7) comprennent au moins un organe parmi :
- un organe lumineux (71) ;
- un organe vibratoire (72) ;
- un organe sonore (73).

7. Système (1) selon la revendication 5 ou 6, **caractérisé en ce que** le dispositif (2) comprend un actionneur (9) relié aux moyens sensoriels (7) et actionnable par l'individu pour désactiver les moyens sensoriels (7).

8. Système (1) selon l'une quelconque des revendications précédentes, **caractérisé en ce que** le dispositif (2) comprend également au moins l'un parmi :
- un capteur (32) de température ;
- un capteur (33) d'oxygène ;
- un accéléromètre (10) ;
- un capteur d'hydratation (34).

## Patentansprüche

1. System (1), umfassend:
- eine Vorrichtung (2), die bestimmt ist, mit dem Körper eines Individuums fest verbunden zu sein, wobei die Vorrichtung (2) Messeinrichtungen (3) umfasst, die mindestens einen Sensor (31) vom Typ Photoplethysmogramm integrieren, der bestimmt ist, Moment-Rohdaten zu erfassen;
- eine erste Berechnungseinheit (41) eines momentanen Pulswellenwerts ausgehend von den Moment-Rohdaten des photoplethysmographischen Sensors (31);
- eine Speichereinheit (5) der berechneten Pulswellenwerte;
- eine Verarbeitungseinheit (42) der gespeicherten Pulswellenwerte, umfassend:
- Identifikationsmittel einer Periode für die gespeicherten Pulswellenwerte;
- eine zweite Berechnungseinheit, um für jede identifizierte Periode zu berechnen:
- eine Amplitudendifferenz und eine Zeitdifferenz zwischen einem ersten Punkt, der einer systolischen Spitze entspricht, und einem zweiten Punkt, der einer diastolischen Spitze entspricht,
- einen Medianmoment zwischen dem ersten Punkt und dem zweiten Punkt,
- eine dritte Berechnungseinheit (43), um ausgehend von der Amplitudendifferenz, von der Zeitdifferenz und von dem Medianmoment mittels einer multivarianten statistischen Analyse schrittweise mit peripherer Kalibrierung die folgenden drei physiologischen Parameter zu berechnen:
- einen arteriellen Rigiditätsindex;
- einen arteriellen Druck;
- einen zentralen Druck,
- eine vierte Berechnungseinheit (44) eines zeitabhängigen Variationswerts der drei physiologischen Parameter zu zwei verschiedenen Momenten T.

2. System (1) nach Anspruch 1, **dadurch gekennzeichnet, dass** es ebenfalls eine externe Kommunikationsvorrichtung (13) vom Typ Smartphone umfasst, die mit der Vorrichtung (2) verbunden ist.

3. System (1) nach Anspruch 1 oder Anspruch 2, **dadurch gekennzeichnet, dass** die Berechnungseinheiten (41, 43, 44), die Verarbeitungseinheit (42) und die Speichereinheit (5) in die Vorrichtung (2) integriert sind.

4. System (1) nach Anspruch 2, **dadurch gekennzeichnet, dass** die Berechnungseinheiten (41, 43, 44), die Verarbeitungseinheit (42) und die Speichereinheit (5) in die externe Kommunikationsvorrichtung (13) integriert sind.

5. System (1) nach vorangehendem Anspruch, **dadurch gekennzeichnet, dass** die Vorrichtung (2) Sensoreinrichtungen (7) umfasst.

6. System (1) nach vorangehendem Anspruch, **dadurch gekennzeichnet, dass** die Sensoreinrichtungen (7) mindestens ein Organ umfasst von:
- einem Leuchtorgan (71);
- einem Vibrationsorgan (72);
- einem Sensororgan (73).

7. System (1) nach Anspruch 5 oder 6, **dadurch gekennzeichnet, dass** die Vorrichtung (2) einen Aktuator (9) umfasst, der mit den Sensoreinrichtungen (7) verbunden und von dem Individuum betätigbar ist, um die Sensoreinrichtungen (7) zu deaktivieren.

8. System (1) nach einem der vorangehenden Ansprüche, **dadurch gekennzeichnet, dass** die Vorrichtung (2) ebenfalls mindestens einen umfasst von:
- einem Temperatursensor (32);
- einem Sauerstoffsensor (33);
- einem Beschleunigungsmesser (10);
- einem Feuchtigkeitssensor (34).

## Claims

1. A system (1) comprising :
- a device (2) intended to be attached to the body of an individual, the device (2) comprising measuring means (3) incorporating at least one sensor (31) of the photoplethysmographic type intended to acquire instantaneous raw data;
- a first unit (41) for calculating an instantaneous pulse wave value from the instantaneous raw data of the photoplethysmographic sensor (31);
- a storage unit (5) for the calculated pulse wave values;
- a processing unit (42) for the stored pulse wave values, comprising:
- means for identifying a period for the stored pulse wave values;
- a second calculation unit to calculate, for each identified period:
- a difference in amplitude and a difference in time between a first point corresponding to a systolic peak and a second point corresponding to a diastolic peak,
- a median moment between the first and second points,
- a third calculation unit (43) for calculating, from the amplitude differrence, the time difference and the median time, by means of a multivariate statistical analysis, step by step with peripheral calibration, the following three physiological parameters:
- an arterial stiffness index;
- blood pressure;
- a central pressure,
- a fourth unit (44) for calculating a time variation value of said three physiological parameters at two different times T.

2. A system (1) according to claim 1, **characterised in that** it also comprises an external communicating device (13) of the smartphone type connected to the device (2).

3. A system (1) according to claim 1 or claim 2, **characterised in that** the computing units (41, 43, 44), the processing unit (42) and the storage unit (5) are integrated in the device (2).

4. A system (1) according to claim 2, **characterised in that** the computing units (41, 43, 44), the processing unit (42) and the storage unit (5) are integrated in the external communicating device (13).

5. A system (1) according to the preceding claim, **characterised in that** the device (2) comprises sensory means (7).

6. A system (1) according to the preceding claim, **characterised in that** the sensory means (7) comprise at least one unit from among:
- a lighting element (71);
- a vibratory unit (72);
- a sound unit (73).

7. A system (1) according to claim 5 or 6, **characterised in that** the device (2) comprises an actuator (9) connected to the sensory means (7) and operable by the individual to deactivate the sensory means (7).

8. A system (1) according to any of the preceding claims, **characterised in that** the device (2) also comprises at least one of:
- a temperature sensor (32);
- an oxygen sensor (33);
- an accelerometer (10);
- a hydration sensor (34).
